# EUROPEAN PATENT SPECIFICATION

(11) **EP 1 834 606 B1**
(45) Date of publication and mention of the grant of the patent: **24.04.2013**
(21) Application number: 06425174.7
(22) Date of filing: 16.03.2006
(51) Int. Cl.: A61F 2/06

(54) **Stents**
Stents
Stents

(43) Date of publication of application: 19.09.2007
(73) Proprietor: CID S.p.A., 13040 Saluggia (VC) (IT)
(72) Inventor: Rolando, Giovanni, 10034 Chivasso (Torino) (IT); Curcio, Maria, 13040 Saluggia (Vercelli) (IT); Grignani, Andrea, 10023 Chieri (Torino) (IT); Gaschino, Paolo, 10090 Castagneto Po (Torino) (IT)
(74) Representative: Bosotti, Luciano

(56) References cited:
- WO-A-2005/118971
- WO-A-2005/120393
- WO-A-2006/060534
- DE-A1- 10 223 399
- US-A1- 2003 055 484

## Description

### Field of the invention

The invention relates to stents: This term in general indicates expandable endo-prostheses capable of being implanted into a lumen in a human or animal body, such as for example a blood vessel, to re-establish and/or maintain its patency.

Stents usually take the form of tubular devices that operate so as to maintain a segment of the blood vessel or other anatomical lumen open. Over recent years, stents have become established for use to treat stenoses of arterioschlerotic nature in blood vessels such as the coronary arteries. The field of application is now gradually extending to other districts and regions of the body, including the peripheral regions.

### Description of the relative technique

The scientific and technical literature concerning stents, including that concerning patents, is very extensive. With regard solely to documents held by the present applicant, we may quote EP-A-0 806 190, EP-A-0 850 604, EP-A-0 875 215, EP-A-0 895 759, EP-A-0 895 760, EP-A-1 080 738, EP-A-1 088 528, EP-A-1 103 234, EP-A-1 174 098, EP-A-1 212 986, EP-A-1 277 449, EP-A-1 449 546, as well as European Patent application 05001267.3.

In this field, a line of research has aimed specifically at producing stents of the biodegradable type (for example of bio-erodible or bio-absorbable material): in other words, these are stents made of materials (for example using polymers but also metals or alloys) such that, after implantation of the stent, they undergo degradation that in practice causes the disappearance of the stent. Examples of this line of research include EP-A-0 554 082 and EP-A-0 894 505.

The development of biodegradable stents takes as its starting point the following consideration: it is known that, after implantation of a stent, the risk that the treated vessel undergo re-stenosis, if this is to occur, exists in the first 6-12 months, whereas the risk of this happening in the longer term is very small indeed. From the biological standpoint the explanation -- as far as is known at present -- is that re-stenosis is caused by a series of factors linked chronologically to implantation of the stent. If during the time span indicated these factors are overcome, this means that the lesion of the blood vessel has healed and, in practical terms, there is no longer any need to have a stent present that maintains patency. Hence the idea of producing a stent that, having completed its function, disappears from the treated blood vessel, eliminating the presence of what is always a foreign body.

Apart from the conceptual interest, now studied for many years, the most evident obstacle to be overcome in producing a stent of biodegradable material lies in the fact that, in order to have adequate radial strength, comparable to that of traditional stents, the structure must be of a thickness that compromises its basic functional aspects (ease of implantation, etc.) and that causes problems of safety (risk of thrombosis due to turbulence). Furthermore, biodegradable materials such as bio-erodible polymers are in general known to cause inflammatory conditions, harbinger of re-stenosis: to implant such a mass of these polymers as is needed to guarantee the required initial strength may lead to serious problems of biocompatibility.

Biodegradable stents of a metallic type (based on corrosible metals, such as for example magnesium) are less widespread. Indeed, the scientific community has up to now been concerned about the expediency of having a rapid and massive local release of metal ions, resulting from the corrosion, and about the true predictability of the time span during which the mechanical strength is lost, and on the progression of the phenomenon.

Independent of all other considerations (choice of materials, kinetics of erosion or absorption, etc.) stents of the biodegradable type must come to terms with a basic problem: before it is fully biodegraded, the stent (or better what remains of the stent as it undergoes gradual degradation) constitutes a sort of "remnant" that can undergo deformation or even dislocation from the site of implantation. These phenomena may be dangerous, for example because they can cause occlusion of the treated blood vessel or can trigger the formation of thrombi.

Research concerning stents has gradually widened to include other details of production, and in particular to the sector of the so-called "drug eluting" stents (DES); this field deals with the possibility of applying onto the stent, or otherwise associating to the stent, substances having the nature of a drug, thus capable of exercising specific activity at the stent implantation site. This in particular with regard to the possibility of associating to the stent drugs with action antagonistic to re-stenosis.

For example, EP-A-0 850 604 describes the possibility of providing stents with sculpturing comprising, for example, cavities capable of receiving one or more drugs useful for the prevention or treatment of re-stenosis and/or substances appropriate for correct use of the stent (adhesion, release modalities, kinetics, etc.). This surface sculpturing is characterised both by the shape and surface area of the cavity, and by its in-depth profile. For example, the cavities may be cavities with circular openings or oval-shaped openings or again elongated openings. Alternatively, they may take the form of an appropriate alternation of cavities with openings of different types depending on the release requirements. The in-depth profile may be "U" or "V" shaped, or again in the form of a vessel with or without a superficial part entirely dedicated to receiving the substances of interest indicated above. This superficial part may have the aspect of a sort of continuous layer only on the outer surface of the stent.

A great deal of work has been dedicated over recent years to identifying the nature of the material -- and in particular of the drug -- loaded onto the stent. This may consist of a single drug, a pair of drugs, or a series of drugs with similar, synergistic or diversified action. Alongside pharmacologically-active molecules, the stent may also carry substances functioning as adjuvants to the pharmacologically-active substances, such as polymers or excipients of various types. The function may be to stabilise the active principle or principles, or may be finalised to regulating release kinetics (slowing or accelerating release). The polymers/excipients may be mixed with the drug or drugs, or may be in separate layers with respect to the pharmacologically-active substances, for example forming a sort of stopper of biodegradable polymer over the hollow or alternatively creating a stratified structure with successive layers of drug and polymer.

Although this type of application is not at present considered particularly attractive among the scientific community, the activity of substances loaded onto the stent may be linked to the fact of their having radioactive nature.

Also in regard to these aspects, the technical and scientific literature and that concerning patents is very extensive, as is shown -- as well as by some of the documents already quoted -- by others such as, for example, EP-A-0 551 182, EP-A-0 747 069, EP-A-0 950 386, EP-A-0 970 711, EP-A-1 254 673, EP-A-1 254 674, WO-A-01/87368, WO-A-02/26280, WO-A-02/26281, WO-A-02/47739, WO-A-02/056790 and again WO-A-02/065947 as well as the literature quoted in these documents; documents and literature that, be it understood, do not in any way exhaust the body of literature on the subject.

With regard to the choice of drug with functions antagonistic to re-stenosis, drugs known as rapamycin (sirolimus) and FK506 (tacrolimus) have taken on particular importance.

The problems connected to the use of drugs on the stent are not, however, limited to the choice of drug alone, in other words to the identification of the substance or substances used, but also involve several further aspects. Among these further aspects, for example, we may mention:
-- the physical form of the substance to be loaded,
-- the loading technique of the material,
-- the technique for cleaning off excess material deposited, and
-- stabilisation of the material.

The loading techniques must evidently take into account the nature (that is the physical form) of the substance or substances to be loaded onto the stent.

Some loading techniques of known type essentially operate in an indirect fashion, since they substantially entail applying a coating onto the stent, typically of polymeric material (for example polymers of metacrylate, polyurethane, PTFE, hydrogel or mixtures of hydrogel/polyurethane, especially PTFE), to or in which the drug to be applied onto the stent is bonded and/or dissolved before application of the coating, destined then to be stabilised by polymerisation.

Other techniques on the contrary substantially entail starting from agents in liquid form or from solutions or dispersions with low viscosity.

This above all in consideration of the fact that, in most cases considered, the drugs of interest are substances that -- originally, or in the form in which they are available in commerce -- are in the form of powders (with different granulometry).

The simplest solution entails loading the stent by immersing it in a vector, typically a liquid, in which is dissolved, suspended or in any case present the substance or substances to be loaded onto the stent. This technique, which may also if necessary be done under vacuum, is known in the art as "dipping".

For example, a solution is described in the document WO-A-02/065947 in which the stent is brought into contact with a solution of FK506 in an aqueous or organic solvent (typically in alcohol, such as ethanol, at a concentration of 3.3 mg of FK506 in 1 ml of ethanol). This, for example, comes about through dripping, spraying or immersing, for preference under vacuum. The stent is then dried, preferably until the solvent is eliminated, and the operation is repeated from 1 to 5 times. Subsequently the stent is if necessary washed once or more than once with water or isotonic saline solution, finally being dried.

To complete the overview of the background of the present invention, it must be mentioned that from the first developments of stent technology (see for example EP-A-0 540 290) it has been very clear to technicians that the characteristics of longitudinal flexibility of a stent come into play in two different contexts:
-- when the stent, arranged in its radially-contracted condition on the implantation catheter, is advanced through the patient's vascular system until it reaches the implantation site (so-called "trackability"), and
-- when the stent, implanted in its radially-expanded condition at the treatment site and after the implantation catheter has been removed, must correctly maintain its implanted position at a vascular site subject to cyclic deformation under the action of the pulsating blood flow and/or that of the cardiac mass that contracts rhythmically, without altering the natural "compliance" of the blood vessel.

More specifically, the invention relates to a stent according to the preamble of Claim 1, which is known e.g. from DE 102 23 399 A1. A somewhat similar arrangement is known from WO 2005/118971 A.

### Object and summary of the invention

The invention aims to take into account a series of essential factors that have to date been linked in a more or less indissoluble fashion to the production of stents of the "drug eluting" type, and that is:
-- the complexity of the operation of loading the drug or active principle,
-- the need, where a coating is produced on the stent, in which the drug to be applied to the stent is bonded and/or dissolved, to take into account the characteristics of the coating, also with regard to the possible subsequent elimination of the coating itself,
-- the difficulty of achieving selective coatings, that is coatings limited to circumscribed areas of the stent,
-- the objective difficulty of loading a plurality of different agents with a limited number of stages, and
-- the critical aspect intrinsically linked to the contemporary loading of more than one agent and if necessary excipients or other substances that can contribute to controlling release kinetics.

The invention has the object of providing a solution that is able to overcome the above difficulties in a radical fashion.

According to the present invention, this object is achieved thanks to a stent having the characteristics indicated specifically in Claim 1. The claims form an integral part of the disclosure provided here in regard to the invention.

In order to achieve its object, the solution described here is based on the concept of stents made of biodegradable material (for example, bio-erodible or bio-absorbable) that is a material that, when exposed to the biological environment in which the stent is implanted (typically a vascular site), undergoes a phenomenon of decay that brings about its gradual disappearance. For the purposes of the present application, the definition of biodegradable material thus leaves completely out of consideration the mechanism (erosion, absorption, corrosion, etc.) that underlies this behaviour.

The solution described here thus concerns, in the presently preferred embodiment, a stent comprising a tubular structure that is selectively expandable between a radially-contracted condition, in which the stent is capable of being carried to the site of implantation, and a radially-expanded condition, in which the stent, positioned at the implantation site, is able to sustain the blood vessel subjected to treatment in an open, patent position, thus eliminating the stenosis; said tubular structure comprises a part of non-biodegradable material and a part of biodegradable material.

In the presently preferred embodiment, the solution described here substantially entails developing what might be called a hybrid stent, comprising:
-- a basic structural part -- made of non-biodegradable material and thus destined to remain at the implant site (thus providing the supporting action to the walls of the treated blood vessel without having a negative effect on the natural feature of "compliance" of the blood vessel) -- typically comprised of a small number of expandable annular elements, connected together or otherwise, that provide the principal radial supporting function; and
-- a part made of biodegradable material, destined to provide, together with the basic structural part, structural coherency and flexibility to the stent when it is implanted, also co-operating in the supporting function (for example local support of the plaque, avoiding prolapse) but destined to disappear some months after implantation, once healing of the treated blood vessel has been achieved.

The solution described here offers a significant contribution to the sector of medicated stents: the part of stent made of biodegradable material represents an excellent drug carrier, from which the drugs can be released slowly over time and, given the masses involved, one that can be loaded with much greater quantities than the devices in current use.

As will be better understood in the detailed description of some exemplary embodiments that follows, the solution described here makes it possible to greatly simplify the operation of loading drug or active principle, making the choice of other components (vectors, excipients, etc.) associated to the drug much less critical. Furthermore, drug loading of the selective type can more easily be achieved, that is loading limited to circumscribed areas of the stent, also in regard to the possible use of a plurality of different agents, also resolving all critical points intrinsically linked to the contemporary loading of more than one agent or if required excipients or other substances capable of contributing to the control of release kinetics.

It will also be understood that the solution described here overcomes the typical demonstrated drawbacks of stents of the biodegradable type. The part of stent that is biodegradable no longer requires to be massive, but can be of dimensions compatible with those of stents in current use. Once the biodegradable part has disappeared, in any case the non-biodegradable basic part of the stent, of itself little invasive, remains solidly and precisely on site.

### Short description of the attached figures

The invention will now be described, by way of nonlimiting example, with reference to the attached drawings, in which:
-- figures 1 and 2 show, in diagram form, the basic part of the stent described here, and
-- figures 3, 4 and 5 correspond to three possible embodiments of the stent described here.

In general, the solution according to the invention lends itself to being produced within the sphere of a stent structure of the type described, for example, in EP-A-0 875 215, comprising:
-- a plurality of annular elements the walls of which follow a looped path (typically sinusoidal or approximately sinusoidal) aligned along the axis of the stent (direction z in the figures) and selectively expandable between a radially-contracted position and a radially-expanded position to achieve the expansion movement of the stent, and
-- a network of longitudinal connecting elements (in general known as "links") that extend like a bridge to connect the annular elements; said connecting elements are in general capable of extending and contracting in the longitudinal direction of the stent (for example by effect of a general λ or Ω conformation, see in this connection EP-A-0 875 215) in order to give the stent the properties of longitudinal flexibility required to guarantee that it displays the appropriate "trackability" during its implantation.

In other words, these are stents in which the dual functions of radial expandability and longitudinal flexibility are, in distinct and separate fashion, provided by two different sets of members, that is the annular elements with looped wall (radial expandability of the stent) and the connecting elements or links (longitudinal flexibility).

From the conceptual standpoint, the solution described here is based on recognition of the fact that the presence of both parts or components of the stent is required, from the structural standpoint, only during the phase of stent implantation (inserting the stent and guiding it towards the implant site employing a catheter, expansion of the stent at the implant site), and that the network of longitudinal connecting elements or links concludes its function during implantation and the immediately subsequent phases (for example, to provide a supporting action of the plaque, avoiding its prolapse inside the treated vessel).

Having completed its function and obtained healing of the treated site, the connecting structure may in fact disappear. Hence the choice, adopted in the solution described here, of producing this part, at least to a substantial extent, of biodegradable material, that is of material destined to disappear during a shorter or longer timeframe (once again it is mentioned that the term "biodegradable" is used here in its widest sense, without specific reference to any mechanism underlying the gradual disappearance of the material itself).

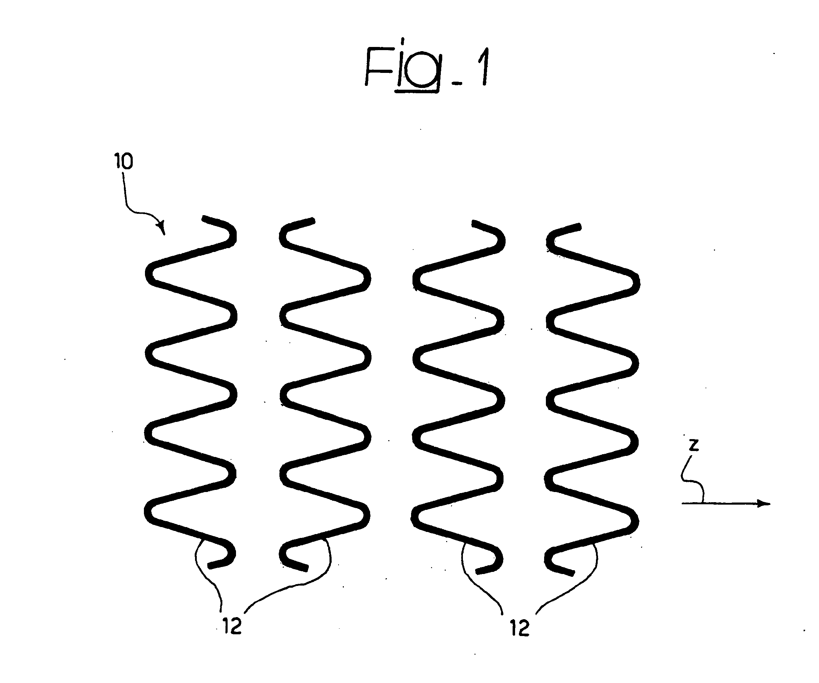

Figure 1 thus shows (in ideal flat development, following the practice generally adopted to represent the structure of stents) the basic part of the stent of the type described here.

In the specific case, this basic part, indicated with 10, is comprised of four annular elements 12 that, with the stent considered in its typical tubular configuration, present an overall cylindrical shape and a looped path. In the specific case, the looped path in question is represented by a sinusoidal trajectory and the various elements 12 are positioned mutually such that their sine waves are in phase opposition. In other words, following figure 1 from left to right, note that, for example, the first and second elements 12 present opposed valleys and peaks (where the first element 1 presents a valley facing towards the second element 12, the second element 12 presents a valley facing towards the first, and so on). In a similar fashion, the second and third elements 12 present opposed valleys and peaks, while the same is also true of the third and fourth elements 12.

The stent according to the invention is in general capable of containing any plural number of elements 12.

From the theoretical standpoint, each of the elements 12 might be seen as representing a stent even when taken singly: nevertheless, the solution described here relates to stents in which a plurality of these elements are present, connected one to another by links or longitudinal connecting elements, whose characteristics will be better described below.

In the solution described here, the part of the structure of the stent comprising the elements 12 is made of a "non-biodegradable" material, that is of durable material of the type normally used to make stents and in general materials are indicated such as stainless steel, cobalt-chromium alloy, etc., if appropriate surface-treated by applying a layer of biocompatible carbonaceous material in the way described, for example, in US-A-4 624 822, US-A-4 758 151, US-A-5 084 151, US-A-5 133 845, US-A-5 370 684, US-A-5 387 247, and US-A-5 423 886.

The term "durable" is thus used in opposition to the term "biodegradable". In substance, the basic part indicated with 10 constitutes, in the solution described here, the part of the stent destined to remain at the implant site in the long term, that is after the parts made of biodegradable material have disappeared.

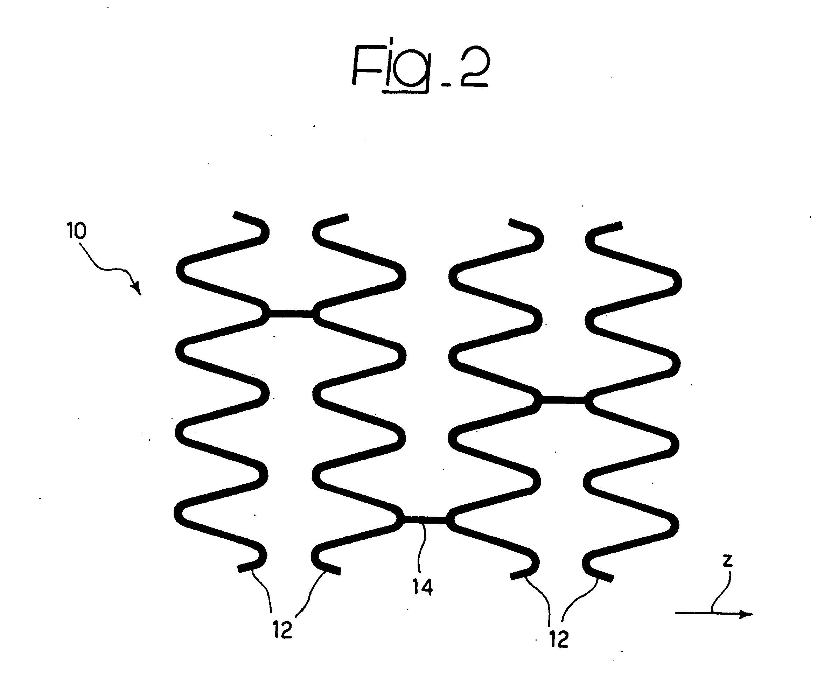

Figure 2 shows that, in some possible embodiments of the solution described here, longitudinal connecting elements 14 may be situated between the elements 12 and may extend in the fashion of a bridge between the elements 12 in the longitudinal direction of the stent, indicated as reference z.

Independently of their extension in the longitudinal sense, axial with regard to the stent, the elements 14 must not be confused with the connecting elements or "links" (indicated on the contrary with 18) destined to give the stent overall the characteristics of longitudinal flexibility.

This is because:
-- the elements 14 are typically made in the form of linear elements ("struts") of fixed length and, as such, are non-extensible. As such, they may not therefore co-operate in any way, in a stent of the type described here, to provide the longitudinal flexibility, which indeed presupposes the fact that the connecting elements or links may vary in length; and
-- in any case the elements 14 -- even if extensible - - are present in a limited number (for example one or two elements 14 placed to connect two adjacent elements 12) and in consequence they only comprises a minor part (less than 50%) and usually a very minor part (no more than 25%, usually less than 20% or less than 10%) of the overall number of elements that extend in the longitudinal direction (z axis) of the stent to link adjacent elements 12.

If present, the elements 14 are usually made of the same material as the elements 12, and usually made as a piece with the elements 12 in the sphere of processing procedures (laser cutting of a micro-tube or hypotubing) normally used to manufacture lasers in current use.

In substance, if present, the elements 14 perform the sole function of avoiding that the individual elements 12 of the basic structure 10 destined to remain in place long-term after implantation of the stunt can undergo undesired phenomena of reciprocal displacement and/or take on an undesired orientation. In other words, the elements 14 essentially act as spacers.

Figures from 3 to 5 illustrate some ways of coupling to a basic structure 10 illustrated in figure 1 a set of connecting elements 18 destined to complete the structure of the stent so as to give the stent the features of mechanical coherency necessary for the implantation stage.

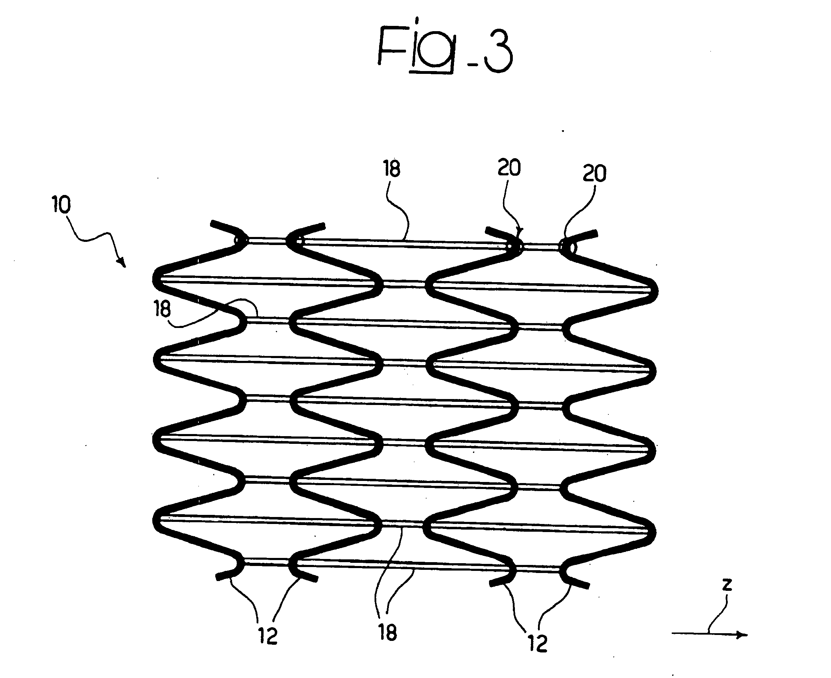

In particular, in the embodiment in figure 3, the connecting elements 18 are comprised of linear bodies (in practice fibres or "spaghetti") of biodegradable polymeric material, preferably associated (in a manner described more clearly below) to at least one active principle such as for example an agent antagonistic to re-stenosis.

The elements 18 are based on a biodegradable material that in general presents characteristics of elasticity, that is longitudinal extensibility. This means that the stent formed of the series of elements 12 and elements 18 is capable of flexing longitudinally along its z axis so as to be able to follow the tortuous path within the treated patient's vascular system along which it advances towards the implant site.

For example, a polymer material presenting the required characteristics may be selected from among:
- polylactic acid;
- poly-ε-caprolactone,
- polyorthoesters,
- polyanhydrides,
- poly-3-hydroxibutyrrate,
- polyaminoacids such as polyglycine,
- polyphosphazenes,
- polyvinyl alcohol,
- low molecular weight polyacrylates,
- copolymers of these.

Among metallic biodegradable materials, iron and magnesium may be used.

These materials lend themselves to being produced in the form of filiform elements such as fibres or spaghetti with circular section presenting a diameter in the order of 0.1 mm.

Their physical characteristics guarantee that the elements 18 will contribute in full to providing the necessary characteristics of mechanical coherency of the stent without undergoing undesired fracture.

At the same time, the material of the type described is able to ensure complete biodegradation (thus in practice the disappearance of the elements 18) within a period of time of the order one to six months after implantation of the stent.

The biodegradable material of the elements 18 lends itself to being loaded with an active principle such as, for example, an agent antagonistic to re-stenosis. Known agents, such as FK506, paclitaxel or rapamycin are some examples of drugs capable of being employed to advantage in the context described here.

In particular, the possibility exists of selecting the (bio)degradation timeframe of the material of the elements 18, in correlation with the time of efficacy of the active principle associated with that material. This in such a fashion that, for example, elements 18 loaded with an active principle of "rapid" effect degrade more rapidly than elements loaded with an active principle of slower action. It will also be understood that the degradation mechanism and kinetics of the elements 18 may be exploited to control release of the active principle.

According to a particularly advantageous aspect of the solution described here, each of the elements 18 is capable of being made of a different material, or at least of being loaded with a different active principle. This in such a fashion as to give the stent overall the structure of a true machine to supply different active principles according to their respective release kinetics.

With regard to the manner in which they are coupled to the biodegradable material, different solutions may be employed.

The active principle may simply be mixed with the biodegradable material that, gradually becoming consumed, provides gradual release of the active principle.

Above all for those active principles for which rapid delivery is preferred, as an acute dosage, it is also possible to design a co-formation or co-extrusion mechanism. This in such a way that the elements 18, as initially provided on the stent, are in reality each comprised of two fibres or spaghetti: one consists of biodegradable material and the other of active principle (or of a vector containing active principle), the two fibres being linked together through a co-extrusion mechanism. Co-extrusion techniques of this type are in current use for example in the production of the so-called "conjugated" polyethylene/polypropylene fibres to produce absorbent mass for sanitary articles.

Naturally, if this solution is employed, it is also possible to vary the type and dosage of active principle along the longitudinal extension of the element 18, such as to be able to release, for example, a first active principle (or a larger quantity , of a specific active principle) in correspondence with the extremities of the stent and a different type of active principle (or a smaller quantity of the same active principle) at the central portion of the same stent.

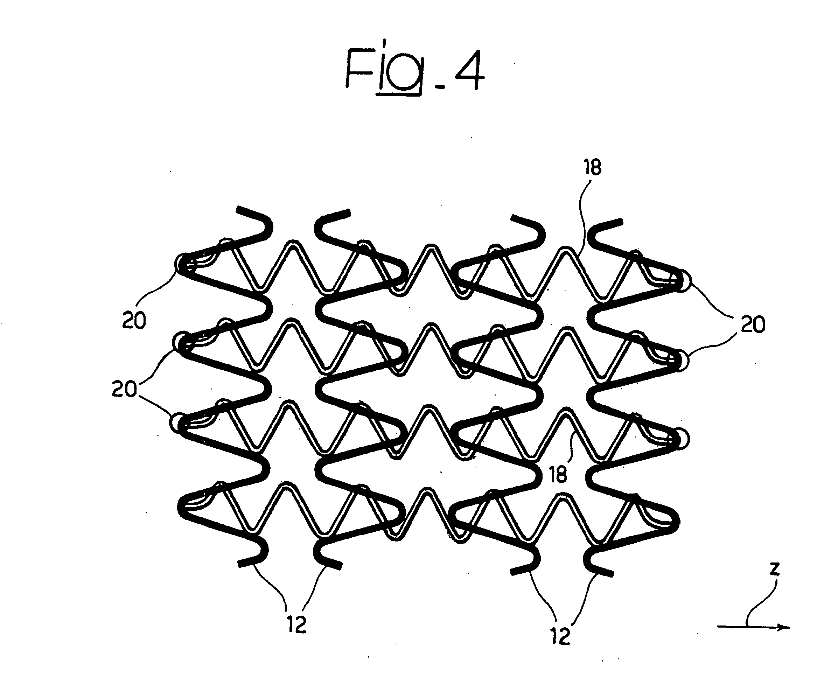

The solution represented in figure 4 essentially corresponds to the solution represented in figure 3, the difference deriving from the fact that, in this case, instead of presenting a straight line the elements 18 are serpentine, for example following a sinusoidal curve. A solution of this type makes it possible to give the stent great longitudinal flexibility without this being translated into corresponding axial traction stresses with regard to the elements 18.

In this case, indeed, the longitudinal flexibility of the stent is chiefly provided by effect of spreading apart the loops in the trajectory followed by the elements 18.

For the variant represented in figure 4 all of the same considerations hold that were made previously with regard, for example, to loading and dosage, as well as the release kinetics of the active principle.

Those of skill in the art will however realise that the solution described (in particular the embodiment in which a large number of elements 18 are present, for example approximately 10, distributed around the peripheral outline of the stent, as in the case of the embodiment represented in figure 3) makes it possible to apply high dosages of active principle onto the stent.

For example, employing the solution represented in figure 3, it may be hypothesised that, onto a stent of normal dimensions, quite a large quantity (for example 1 mg) of agent antagonistic to re-stenosis can be loaded, such as micophenolic acid, rapamycin, tacrolimus, cyclosporin, or corticosteroids.

With regard in particular to the release kinetics of the active principle, it should again be mentioned that elements of an elongated shape such as the elements 18 in figures 3 and 4 lend themselves to acting as vectors for nanoparticles containing an active principle or active principles distributed in a differentiated fashion along the stent. In this connection, an association between fibres of biodegradable material and nanoparticles to which reference may be made is documented in EP-A-1 080 738.

In this connection, experts in the sector will immediately realise that, though there may be some affinity between the solutions illustrated, for example, in figure 4 of the present application, and the solutions illustrated in figures 1 and 2 of EP-A-1 080 738, an essential conceptual difference exists between the solution described here and the solution described in that previous document of known technique. This fundamental difference lies in the fact that, in the solution documented in EP-A-1 080 738, the fibres containing the nanoparticles are superimposed, combined or in some way interwoven onto a basic structure that of itself is a stent. In particular it continues in all respects to be a stent even if the application of such fibres is not intended.

On the contrary, in the solution described here, the structure of the stent is provided solely by the combination (and by the synergistic co-operation) of the elements 12 and the elements 18: the basic structure represented in figures 1 and 2 of the present application is in fact not of itself capable of providing the full functionality of a stent, not of itself being able to ensure the features of mechanical coherency and "trackability" necessary to enable implantation of the stent and required in the phases immediately subsequent to implantation.

To ensure this result in the solution described here, the elements 18 must obviously be connected to the elements 12. This comes about in correspondence with anchorage points 20 located for preference in correspondence with the cusps of the loops of the elements 12. This choice derives from the fact that said cusp zones are not subjected to rotation movements during radial expansion of the stent. For the formation of the anchorage points 20 different solutions may be employed (hot welding, cementing) compatible with the nature of the material comprising the elements 12 and the material comprising the elements 18 in the terms described above. A possible alternative (considered less preferable at present) comprises anchorage by mechanical interlock. This solution may be adopted, for example, when the parts of the cusps of the loops of the elements 18 present an eyelet conformation.

For particular geometric forms of the elements 12 (for example the geometry represented in figure 3 of EP-A-0 875 215) it may also be hypothesised that the connection can come about through weaving, in the sense that the elements 18 are woven around the elements 12 and held in position by effect of the weave trajectory.

It will likewise be understood that, although in the embodiments represented in figures 3 and 4, the elements 18 extend in a practically continuous fashion along the entire longitudinal extension of the stent, a varied embodiment can undoubtedly be hypothesised in which the elements 18 have a lesser extension, for example linking only adjacent elements 12.

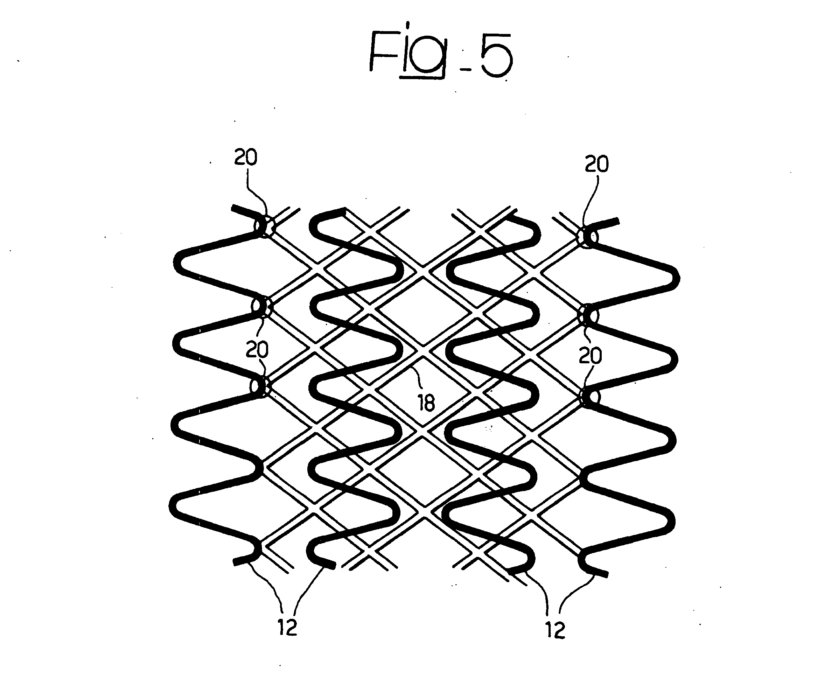

Figure 5 shows another possible alternative embodiment wherein the elements 18 that extend following a sinusoidal trajectory are not produced as dependent elements but as in the form of a network structure of biodegradable material capable of fitting around the basic structure 10 and held there exclusively by elastic forces (although the presence of anchorage or welding points 20, at least in correspondence with the extremities of the stent of the network is undoubtedly to be considered preferable).

Once again, it will not escape the notice of those of skill in the art that, though presenting some similarity with figure 6 in EP-A-1 103 234, the solution described here presents an evident and fundamental basic difference compared to the solution described in that previous document of known technique. Once again, in fact, in the solution documented in EP-A-1 103 234 the basic structure onto which the network is applied is of itself a stent.

It will again be appreciated that the application of a layer of biocompatible carbonaceous material following the modalities described, for example, in US-A-4 624 822, US-A-4 758 151, US-A-5 084 151, US-A-5 133 845, US-A-5 370 684, US-A-5 387 247, and US-A-5 423 886, will for preference involve only the non-biodegradable parts of the stent and will not extend to the biodegradable parts.

Naturally, the principle of the invention holding good, the details of production and the embodiments may be widely varied with regard to what is described and illustrated here, without thereby departing from the sphere of the present invention, as defined by the attached claims. In particular, it will be appreciated that the basic concept of making the tubular structure so that it includes a part of non-biodegradable material and a part of biodegradable material lends itself to embodiments according to Claim 1 in which some of the annular elements 12 are they too made of biodegradable material.

## Claims

1. A stent including a tubular structure (12, 14, 18) selectively expandable between a radially-contracted condition and a radially-expanded condition,
wherein said tubular structure includes a part (12, 14) of non-biodegradable material and a part (12, 18) of biodegradable material,
wherein said tubular structure (12, 14, 18) includes:
-- a plurality of annular elements (12) aligned along the direction of extension of the stent (z), said annular elements (12) being selectively expandable between a radially-contracted condition and a radially-expanded condition, and
-- a series of connecting elements (18) that extend in the direction of extension of the stent (z) to connect said annular elements (12),
**characterized in that** said longitudinal connecting elements (18) are made of biodegradable material and **in that** said plurality of annular elements (12) comprises some annular elements made of biodegradable material and other annular elements made of non-biodegradable material.

2. Stent according to claim 1, **characterised in that** the annular elements (12) made of non-biodegradable material are of metallic material.

3. Stent according to claim 2, **characterised in that** said metallic material is selected out of steel and a cobalt-chromium alloy.

4. Stent according to any of the above claims, **characterised in that** said annular elements (12) extend following a looped trajectory.

5. Stent according to any of the above claims, **characterised in that** said annular elements (12) extend following a sinusoidal trajectory.

6. Stent according to claim 5, **characterised in that** said plurality of adjacent annular elements (12) extend following a sinusoidal trajectory in phase opposition one to the other.

7. Stent according to any of the above claims, **characterised in that** said plurality of annular elements (12) are connected by longitudinal connecting elements (14) of non-biodegradable material.

8. Stent according to claim 7, **characterised in that** said connecting elements of non-biodegradable material are substantially non-extensible in the longitudinal direction (z) of the stent.

9. Stent according to claim 7 or claim 8, **characterised in that** said connecting elements of non-biodegradable material (14) are present, connecting between pairs of said adjacent annular elements, in a lower number than said connecting elements (18) of biodegradable material.

10. Stent according to claim 9, **characterised in that** said connecting elements (14) of non-biodegradable material, connecting pairs of said annular elements (12) adjacent one to the next, are present to an extent not above 25% of said connecting elements (18) of biodegradable material.

11. Stent according to claim 10, **characterised in that** said connecting elements (14) of non-biodegradable material, connecting pairs of said annular elements (12) adjacent one to the next, are present to an extent not above 20% of said connecting elements (18) of biodegradable material.

12. Stent according to claim 11, **characterised in that** said connecting elements (14) of non-biodegradable material, connecting pairs of said annular elements (12) adjacent one to the next, are present to an extent not above 10% of said connecting elements (18) of biodegradable material.

13. Stent according to any of the above claims, **characterised in that** said biodegradable material is a polymer.

14. Stent according to any of the above claims, **characterised in that** said biodegradable material is selected in the group consisting of:
- polylactic acid;
- poly-ε-caprolactone,
- polyorthoesters,
- polyanhydrides,
- poly-3-hydroxibutyrrate,
- polyaminoacids such as polyglycine,
- polyphosphazenes,
- polyvinyl alcohol,
- low molecular weight polyacrylates,
- co-polymers of the above.

15. Stent according to any of the above claims from 1 to 12, **characterised in that** said biodegradable material is selected from iron and magnesium.

16. Stent according to any of the above claims, **characterised in that** said part of biodegradable material includes elements (18) of biodegradable material extending following a substantially straight trajectory in the direction of extension (z) of the stent.

17. Stent according to any of the above claims, **characterised in that** said part of biodegradable material includes elements (18) of biodegradable material that extend following trajectories that are substantially looped in shape, with loops oriented transversally to the direction of extension (z) of the stent.

18. Stent according to claim 17, **characterised in that** said looped trajectories are sinusoidal trajectories.

19. Stent according to any of the above claims, **characterised in that** said part of biodegradable material includes elements (18) of biodegradable material extending over the entire length of the stent.

20. Stent according to any of the above claims, **characterised in that** said part of biodegradable material includes elements (18) of biodegradable material extending over a part of the length of the stent.

21. Stent according to any of the above claims, **characterised in that** anchorage points (20) are provided between said part of biodegradable material (18) and said part of non-biodegradable material (12, 14).

22. Stent according to claim 21, **characterised in that** said anchorage points (20) are welding spots between said non-biodegradable material and said biodegradable material.

23. Stent according to claim 21, **characterised in that** said anchorage points (20) are adhesive points between said non-biodegradable material and said biodegradable material.

24. Stent according to claim 21, **characterised in that** said anchorage points (20) are interweaving points between said longitudinal connecting elements (18) of biodegradable material and said annular elements (12).

25. Stent according to any of the above claims, **characterised in that** said part of biodegradable material (18) forms a network structure that fits over said part of non-biodegradable material (12, 14).

26. Stent according to any of the above claims, **characterised in that** said part of biodegradable material (18) carries a drug.

27. Stent according to claim 26, **characterised in that** said drug is an agent antagonistic to re-stenosis.

28. Stent according to claim 27, **characterised in that** said agent antagonistic to re-stenosis is chosen out of paclitaxel, rapamycin, micophenolic acid, rapamycin, tacrolimus, cyclosporin, and corticosteroids.

29. Stent according to any of the above claims from 26 to 28, **characterised in that** said part of biodegradable material includes a plurality of elements (18) of biodegradable material that carry different drugs one from the other.

30. Stent according to any of the above claims from 26 to 29, **characterised in that** said part of biodegradable material includes a plurality of elements (18) of biodegradable material that carry different dosages of the same drug.

31. Stent according to any of the above claims from 26 to 30, **characterised in that** said part of biodegradable material includes at least one element (18) of biodegradable material that carries different drugs along the longitudinal development of the stent.

32. Stent according to any of the above claims from 26 to 31, **characterised in that** said part of biodegradable material includes at least one element (18) of biodegradable material that carries different dosages of one and the same drug along the longitudinal development of the stent.

33. Stent according to any of the claims from 26 to 32, **characterised in that** said drug is mixed with said biodegradable material.

34. Stent according to any of the claims from 26 to 33, **characterised in that** said drug is applied onto said biodegradable material.

35. Stent according to claim 34, **characterised in that** said biodegradable material is in the form of fibres and said drug is co-extruded with said fibres.

36. Stent according to any of the claims from 26 to 35, **characterised in that** said drug is disposed on said part of biodegradable material (18) in the form of nanoparticles.

37. Stent according to any of the above claims, **characterised in that** it includes a coating of biocompatible carbonaceous material applied on said part of non-biodegradable material (12, 14).

38. Stent according to claim 37, **characterised in that** said part of biodegradable material (18) is exempt from said coating of biocompatible carbonaceous material.

## Patentansprüche

1. Stent mit einer röhrenförmigen Struktur (12, 14, 18), die zwischen einem radial zusammengezogenen Zustand und einem radial expandierten Zustand selektiv expandierbar ist,
wobei die röhrenförmige Struktur einen Teil (12, 14) aus nicht-biodegradierbarem Material und einen Teil (12, 18) aus biodegradierbarem Material aufweist,
wobei die röhrenförmige Struktur (12, 14, 18) umfasst:
- eine Vielzahl von ringförmigen Elementen (12), die entlang der Ausdehnungsrichtung (z) des Stents ausgerichtet sind, wobei die ringförmigen Elemente (12) zwischen einem radial zusammengezogenen Zustand und einem radial expandierten Zustand selektiv expandierbar sind, und
- eine Reihe von Verbindungselementen (18), die sich in Ausdehnungsrichtung (z) des Stents erstrecken, um die ringförmigen Elemente (12) zu verbinden,
**dadurch gekennzeichnet, dass** die longitudinalen Verbindungselemente (18) aus einem biodegradierbaren Material gefertigt sind und dass die Vielzahl ringförmiger Elemente (12) einige ringförmige Elemente aus biodegradierbarem Material und andere ringförmige Elemente aus nicht-biodegradierbarem Material umfasst.

2. Stent nach Anspruch 1, **dadurch gekennzeichnet, dass** die ringförmigen Elemente (12) aus nicht-biodegradierbarem Material aus metallischem Material gefertigt sind.

3. Stent nach Anspruch 2, **dadurch gekennzeichnet, dass** das metallische Material ausgesucht ist aus Stahl und einer Kobalt-Chrom-Legierung.

4. Stent nach einem der vorhergehenden Ansprüche, **dadurch gekennzeichnet, dass** sich die ringförmigen Elemente (12) einer schlaufenförmigen Trajektorie folgend ausdehnen.

5. Stent nach einem der vorhergehenden Ansprüche, **dadurch gekennzeichnet, dass** die ringförmigen Elemente (12) sich einer sinusförmigen Trajektorie folgend ausdehnen.

6. Stent nach Anspruch 5, **dadurch gekennzeichnet, dass** die Vielzahl benachbarter ringförmiger Elemente (12) sich einer sinusförmigen Trajektorie folgend gegenphasig zueinander erstrecken.

7. Stent nach einem der vorhergehenden Ansprüche, **dadurch gekennzeichnet, dass** die Vielzahl ringförmiger Elemente (12) durch longitudinale Verbindungselemente (14) aus nicht-biodegradierbarem Material verbunden sind.

8. Stent nach Anspruch 7, **dadurch gekennzeichnet, dass** die Verbindungselemente aus nicht-biodegradierbarem Material im Wesentlichen nicht dehnbar in Längsrichtung (z) des Stents sind.

9. Stent nach Anspruch 7 oder Anspruch 8, **dadurch gekennzeichnet, dass** die Verbindungselemente aus nicht-biodegradierbarem Material (14), die zwischen Paaren benachbarter ringförmiger Elemente verbinden, in einer geringeren Anzahl vorhanden sind als die Verbindungselemente (18) aus biodegradierbarem Material.

10. Stent nach Anspruch 9, **dadurch gekennzeichnet, dass** die Verbindungselemente (14) aus nicht-biodegradierbarem Material, die Paare zueinander benachbarter ringförmiger Elemente (12) verbinden, in einem Ausmaß nicht oberhalb von 25% der Verbindungselemente (18) aus biodegradierbarem Material vorhanden sind.

11. Stent nach Anspruch 10, **dadurch gekennzeichnet, dass** die Verbindungselemente (14) aus nicht-biodegradierbarem Material, die Paare zueinander benachbarter ringförmiger Elemente (12) verbinden, in einem Ausmaß nicht oberhalb von 20% der Verbindungselemente (18) aus biodegradierbarem Material vorhanden sind.

12. Stent nach Anspruch 11, **dadurch gekennzeichnet, dass** die Verbindungselemente (14) aus nicht-biodegradierbarem Material, die Paare zueinander benachbarter ringförmiger Elemente (12) verbinden, in einem Ausmaß nicht oberhalb von 10% der Verbindungselemente (18) aus biodegradierbarem Material vorhanden sind.

13. Stent nach einem der vorhergehenden Ansprüche, **dadurch gekennzeichnet, dass** das biodegradierbare Material ein Polymer ist.

14. Stent nach einem der vorhergehenden Ansprüche, **dadurch gekennzeichnet, dass** das biodegradierbare Material ausgesucht ist aus der Gruppe bestehend aus:
- Polymilchsäure;
- Poly-ε-Caprolacton,
- Polyorthoestern,
- Polyanhydriden,
- Poly-3-Hydroxibutyrrate,
- Polyaminosäuren wie Polyglycin,
- Polyphosphazenen,
- Polyvinylalkohol,
- Polyacrylaten geringen Molekulargewichts,
- Co-Polymere der vorstehenden.

15. Stent nach einem der Ansprüche 1 bis 12, **dadurch gekennzeichnet, dass** das biodegradierbare Material ausgesucht ist aus Eisen und Magnesium.

16. Stent nach einem der vorhergehenden Ansprüche, **dadurch gekennzeichnet, dass** der Teil aus biodegradierbarem Material Elemente (18) aus biodegradierbarem Material umfasst, die sich einer im Wesentlichen geraden Trajektorie in Ausdehnungsrichtung (z) des Stents folgend erstrecken.

17. Stent nach einem der vorhergehenden Ansprüche, **dadurch gekennzeichnet, dass** der Teil aus biodegradierbarem Material Elemente (18) aus biodegradierbarem Material umfasst, die sich im Wesentlichen schlaufenförmigen Trajektorien, mit Schlaufen, die transversal zu der Ausdehnungsrichtung (z) des Stents orientiert sind, folgend erstrecken.

18. Stent nach Anspruch 17, **dadurch gekennzeichnet, dass** die schlaufenförmigen Trajektorien sinusförmige Trajektorien sind.

19. Stent nach einem der vorhergehenden Ansprüche, **dadurch gekennzeichnet, dass** der Teil aus biodegradierbarem Material Elemente (18) aus biodegradierbarem Material umfasst, die sich über die gesamte Länge des Stents erstrecken.

20. Stent nach einem der vorhergehenden Ansprüche, **dadurch gekennzeichnet, dass** der Teil aus biodegradierbarem Material Elemente (18) aus biodegradierbarem Material umfasst, die sich über einen Teil der Länge des Stents erstrecken.

21. Stent nach einem der vorhergehenden Ansprüche, **dadurch gekennzeichnet, dass** Verankerungspunkte (20) zwischen dem Teil aus biodegradierbarem Material (18) und dem Teil aus nicht-biodegradierbarem Material (12, 14) vorgesehen sind.

22. Stent nach Anspruch 21, **dadurch gekennzeichnet, dass** die Verankerungspunkte (20) Schweißpunkte zwischen dem nicht-biodegradierbaren Material und dem biodegradierbaren Material sind.

23. Stent nach Anspruch 21, **dadurch gekennzeichnet, dass** die Verankerungspunkte (20) Haftpunkte zwischen dem nicht-biodegradierbaren Material und dem biodegradierbaren Material sind.

24. Stent nach Anspruch 21, **dadurch gekennzeichnet, dass** die Verankerungspunkte (20) Verflechtungspunkte zwischen den longitudinalen Verbindungselementen (18) aus biodegradierbarem Material und den ringförmigen Elementen (12) sind.

25. Stent nach einem der vorhergehenden Ansprüche, **dadurch gekennzeichnet, dass** der Teil aus biodegradierbarem Material (18) eine Netzwerkstruktur bildet, die über den Teil aus nicht-biodegradierbarem Material (12, 14) passt.

26. Stent nach einem der vorhergehenden Ansprüche, **dadurch gekennzeichnet, dass** der Teil aus biodegradierbarem Material (18) ein Arzneimittel trägt.

27. Stent nach Anspruch 26, **dadurch gekennzeichnet, dass** das Arzneimittel ein Mittel ist, welches Restenose entgegenwirkt.

28. Stent nach Anspruch 27, **dadurch gekennzeichnet, dass** das Mittel, welches Restenose entgegenwirkt, ausgesucht ist aus Paclitaxel, Rapamycin, Micophenolsäure, Rapamycin, Tacrolimus, Cyclosporin und Corticosteroiden.

29. Stent nach einem der Ansprüche 26 bis 28, **dadurch gekennzeichnet, dass** der Teil aus biodegradierbarem Material eine Vielzahl von Elementen (18) aus biodegradierbarem Material umfasst, die unterschiedliche Arzneimittel tragen.

30. Stent nach einem der Ansprüche 26 bis 29, **dadurch gekennzeichnet, dass** der Teil aus biodegradierbarem Material eine Vielzahl von Elementen (18) aus biodegradierbarem Material umfasst, die unterschiedliche Dosen des gleichen Arzneimittels tragen.

31. Stent nach einem der Ansprüche 26 bis 30, **dadurch gekennzeichnet, dass** der Teil aus biodegradierbarem Material wenigstens ein Element (18) aus biodegradierbarem Material umfasst, welches entlang des longitudinalen Verlaufs des Stents unterschiedliche Arzneimittel trägt.

32. Stent nach einem der Ansprüche 26 bis 31, **dadurch gekennzeichnet, dass** der Teil aus biodegradierbarem Material wenigstens ein Element (18) aus biodegradierbarem Material umfasst, das entlang des longitudinalen Verlaufs des Stents unterschiedliche Dosen des gleichen Arzneimittels trägt.

33. Stent nach einem der Ansprüche 26 bis 32, **dadurch gekennzeichnet, dass** das Arzneimittel mit dem biodegradierbaren Material gemischt ist.

34. Stent nach einem der Ansprüche 26 bis 33, **dadurch gekennzeichnet, dass** das Arzneimittel auf das biodegradierbare Material aufgetragen ist.

35. Stent nach Anspruch 34, **dadurch gekennzeichnet, dass** das biodegradierbare Material in Form von Fasern ausgebildet ist und das Arzneimittel mit den Fasern koextrudiert ist.

36. Stent nach einem der Ansprüche 26 bis 35, **dadurch gekennzeichnet, dass** das Arzneimittel in Form von Nanopartikeln auf dem Teil aus biodegradierbarem Material (18) vorgesehen ist.

37. Stent nach einem der vorhergehenden Ansprüche, **dadurch gekennzeichnet, dass** der Stent eine Beschichtung aus biokompatiblem kohlenstoffhaltigen Material umfasst, die auf den Teil aus nicht-biodegradierbarem Material (12, 14) aufgetragen ist.

38. Stent nach Anspruch 37, **dadurch gekennzeichnet, dass** der Teil aus biodegradierbarem Material (18) frei von der Beschichtung aus biokompatiblem kohlenstoffhaltigen Material ist.

## Revendications

1. Stent comprenant une structure tubulaire (12, 14, 18) sélectivement expansible entre une condition radialement contractée et une condition radialement déployée,
dans lequel ladite structure tubulaire comprend une partie (12, 14) en matériau non biodégradable et une partie (12, 18) en matériau biodégradable,
dans lequel ladite structure tubulaire (12, 14, 18) comprend :
une pluralité d'éléments annulaires (12) alignés le long de la direction d'extension du stent (z), lesdits éléments annulaires (12) étant sélectivement expansible entre une condition radialement contractée et une condition radialement déployée, et
une série d'éléments de raccordement (18) qui s'étendent dans la direction d'extension du stent (z) pour raccorder lesdits éléments annulaires (12),
**caractérisé en ce que** lesdits éléments de raccordement longitudinaux (18) sont réalisés dans un matériau biodégradable et **en ce que** ladite pluralité d'éléments annulaires (12) comprend certains éléments annulaires réalisés dans un matériau biodégradable et d'autres éléments annulaires réalisés dans un matériau non biodégradable.

2. Stent selon la revendication 1, **caractérisé en ce que** les éléments annulaires (12) réalisés dans un matériau non biodégradable sont réalisés avec un matériau métallique.

3. Stent selon la revendication 2, **caractérisé en ce que** ledit matériau métallique est choisi parmi l'acier et un alliage de cobalt-chrome.

4. Stent selon l'une quelconque des revendications précédentes, **caractérisé en ce que** lesdits éléments annulaires (12) s'étendent en suivant une trajectoire en boucle.

5. Stent selon l'une quelconque des revendications précédentes, **caractérisé en ce que** lesdits éléments annulaires (12) s'étendent en suivant une trajectoire sinusoïdale.

6. Stent selon la revendication 5, **caractérisé en ce que** ladite pluralité d'éléments annulaires (12) adjacents s'étendent en suivant une trajectoire sinusoïdale en opposition de phase les uns par rapport aux autres.

7. Stent selon l'une quelconque des revendications précédentes, **caractérisé en ce que** ladite pluralité d'éléments annulaires (12) sont raccordés par des éléments de raccordement longitudinaux (14) en matériau non biodégradable.

8. Stent selon la revendication 7, **caractérisé en ce que** lesdits éléments de raccordement en matériau non biodégradable sont sensiblement non extensibles dans la direction longitudinale (z) du stent.

9. Stent selon la revendication 7 ou 8, **caractérisé en ce que** lesdits éléments de raccordement en matériau non biodégradable (14), raccordant entre les paires desdits éléments annulaires adjacents, sont présents en un nombre inférieur auxdits éléments de raccordement (18) en matériau biodégradable.

10. Stent selon la revendication 9, **caractérisé en ce que** lesdits éléments de raccordement (14) en matériau non biodégradable, raccordant des paires desdits éléments annulaires (12) adjacents l'un à l'autre, sont présents jusqu'à un point non supérieur à 25 % desdits éléments de raccordement (18) en matériau biodégradable.

11. Stent selon la revendication 10, **caractérisé en ce que** lesdits éléments de raccordement (14) en matériau non biodégradable, raccordant des paires desdits éléments annulaires (12) adjacents l'un à l'autre, sont présents jusqu'à un point non supérieur à 20 % desdits éléments de raccordement (18) en matériau biodégradable.

12. Stent selon la revendication 11, **caractérisé en ce que** lesdits éléments de raccordement (14) en matériau non biodégradable, raccordant des paires desdits éléments annulaires (12) adjacents l'un à l'autre, sont présents jusqu'à un point non supérieur à 10 % desdits éléments de raccordement (18) en matériau biodégradable.

13. Stent selon l'une quelconque des revendications précédentes, **caractérisé en ce que** ledit matériau biodégradable est un polymère.

14. Stent selon l'une quelconque des revendications précédentes, **caractérisé en ce que** ledit matériau biodégradable est choisi dans le groupe comprenant :
l'acide polylactique ;
le poly-ε-caprolactone,
les polyorthoesters,
les polyanhydrides,
le poly-3-hydroxybutyrate,
les acides polyaminés tels que la polyglycine,
les polyphosphazènes,
l'alcool polyvinylique,
les polyacrylates à faible poids moléculaire,
les copolymères des éléments ci-dessus.

15. Stent selon l'une quelconque des précédentes revendications 1 à 12, **caractérisé en ce que** ledit matériau biodégradable est choisi parmi le fer et le magnésium.

16. Stent selon l'une quelconque des revendications précédentes, **caractérisé en ce que** ladite partie en matériau biodégradable comprend des éléments (18) en matériau biodégradable s'étendant en suivant une trajectoire sensiblement droite dans la direction d'extension (z) du stent.

17. Stent selon l'une quelconque des revendications précédentes, **caractérisé en ce que** ladite partie en matériau biodégradable comprend des éléments (18) en matériau biodégradable qui s'étendent en suivant des trajectoires qui sont sensiblement en forme de boucle, avec des boucles orientées de manière transversale par rapport à la direction d'extension (z) du stent.

18. Stent selon la revendication 17, **caractérisé en ce que** lesdites trajectoires en boucle sont des trajectoires sinusoïdales.

19. Stent selon l'une quelconque des revendications précédentes, **caractérisé en ce que** ladite partie en matériau biodégradable comprend des éléments (18) en matériau biodégradable s'étendant sur toute la longueur du stent.

20. Stent selon l'une quelconque des revendications précédentes, **caractérisé en ce que** ladite partie en matériau biodégradable comprend des éléments (18) en matériau biodégradable s'étendant sur une partie de la longueur du stent.

21. Stent selon l'une quelconque des revendications précédentes, **caractérisé en ce que** des points d'ancrage (20) sont prévus entre ladite partie en matériau biodégradable (18) et ladite partie en matériau non biodégradable (12, 14).

22. Stent selon la revendication 21, **caractérisé en ce que** lesdits points d'ancrage (20) sont des points de soudage entre ledit matériau non biodégradable et ledit matériau biodégradable.

23. Stent selon la revendication 21, **caractérisé en ce que** lesdits points d'ancrage (20) sont des points adhésifs entre ledit matériau non biodégradable et ledit matériau biodégradable.

24. Stent selon la revendication 21, **caractérisé en ce que** lesdits points d'ancrage (20) sont des points d'entrelacement entre lesdits éléments de raccordement longitudinaux (18) en matériau biodégradable et lesdits éléments annulaires (12).

25. Stent selon l'une quelconque des revendications précédentes, **caractérisé en ce que** ladite partie en matériau biodégradable (18) forme une structure de filet qui s'adapte sur ladite partie en matériau non biodégradable (12, 14).

26. Stent selon l'une quelconque des revendications précédentes, **caractérisé en ce que** ladite partie en matériau biodégradable (18) comporte un médicament.

27. Stent selon la revendication 26, **caractérisé en ce que** ledit médicament est un agent antagoniste de la resténose.

28. Stent selon la revendication 27, **caractérisé en ce que** ledit agent antagoniste de la resténose est choisi parmi le paclitaxel, la rapamycine, l'acide mycophénolique, la rapamycine, le tacrolimus, la cyclosporine et les corticoïdes.

29. Stent selon l'une quelconque des précédentes revendications 26 à 28, **caractérisé en ce que** ladite partie en matériau biodégradable comprend une pluralité d'éléments (18) en matériau biodégradable qui comportent des médicaments différents les uns des autres.

30. Stent selon l'une quelconque des précédentes revendications 26 à 29, **caractérisé en ce que** ladite partie en matériau biodégradable comprend une pluralité d'éléments (18) en matériau biodégradable qui comportent différents dosages du même médicament.

31. Stent selon l'une quelconque des précédentes revendications 26 à 30, **caractérisé en ce que** ladite partie en matériau biodégradable comprend au moins un élément (18) en matériau biodégradable qui comporte différents médicaments le long du développement longitudinal du stent.

32. Stent selon l'une quelconque des précédentes revendications 26 à 31, **caractérisé en ce que** ladite partie en matériau biodégradable comprend au moins un élément (18) en matériau biodégradable qui comporte différents dosages d'un seul et même médicament le long du développement longitudinal du stent.

33. Stent selon l'une quelconque des revendications 26 à 32, **caractérisé en ce que** ledit médicament est mélangé audit matériau biodégradable.

34. Stent selon l'une quelconque des revendications 26 à 33, **caractérisé en ce que** ledit médicament est appliqué sur ledit matériau biodégradable.

35. Stent selon la revendication 34, **caractérisé en ce que** ledit matériau biodégradable se présente sous la forme de fibres et ledit médicament est co-extrudé avec lesdites fibres.

36. Stent selon l'une quelconque des revendications 26 à 35, **caractérisé en ce que** ledit médicament est disposé sur ladite partie en matériau biodégradable (18) sous la forme de nanoparticules.

37. Stent selon l'une quelconque des revendications précédentes, **caractérisé en ce qu'**il comprend un revêtement de matière carbonée biocompatible appliqué sur ladite partie en matériau non biodégradable (12, 14).

38. Stent selon la revendication 37, **caractérisé en ce que** ladite partie en matériau biodégradable (18) est dépourvue dudit revêtement de matière carbonée biocompatible.
